# EUROPÄISCHE PATENTSCHRIFT

(11) **EP 1 431 271 B1**
(45) Veröffentlichungstag und Bekanntmachung des Hinweises auf die Patenterteilung: **19.11.2008**
(21) Anmeldenummer: 03026511.0
(22) Anmeldetag: 18.11.2003
(51) Int. Cl.: C07C 209/16, C07C 209/26

(54) **Verfahren zur Herstellung eines Amins**
Process for the preparation of an amine
Procédé pour la préparation d'un amine

(30) Priorität: 20.12.2002 DE 10261193
(43) Veröffentlichungstag der Anmeldung: 23.06.2004
(73) Patentinhaber: BASF SE, 67056 Ludwigshafen (DE)
(72) Erfinder: Gerlach, Till, Dr., 67071 Ludwigshafen (DE); Funke, Frank, Dr., 67067 Ludwigshafen (DE); Melder, Johann-Peter, Dr., 67459 Böhl-Iggelheim (DE)

(56) Entgegenhaltungen:
- EP-A- 0 382 049
- EP-A- 1 020 424
- EP-A- 1 106 600
- EP-A- 1 106 601

## Beschreibung

Die vorliegende Erfindung betrifft ein Verfahren zur Herstellung eines Amins durch Umsetzung eines primären oder sekundären Alkohols, Aldehyds oder Ketons mit Wasserstoff und einer Stickstoffverbindung, ausgewählt aus der Gruppe Ammoniak, primäre und sekundäre Amine, in Gegenwart eines Katalysators.

DE-A1-26 18 580 (BASF AG) beschreibt ein Verfahren zur Herstellung von tertiären Aminen in Gegenwart eines im Wesentlichen trägerfreien Katalysators, der wenigstens 60 % Co und etwa 10 bis 30 % Cu enthält, wobei bis zu 20 % des Kobalts durch Ni ersetzt sein kann.

EP-A2-514 692 (BASF AG) offenbart ein Verfahren zur Herstellung von Aminen aus Alkoholen in Gegenwart Kupfer und Nickel und Zirkonium- und/oder Aluminiumoxid enthaltender Katalysatoren.

EP-A1-382 049 (BASF AG) betrifft Katalysatoren enthaltend Zirkoniumoxid, Kupfer, Kobalt und Nickel und ihre Verwendung in Verfahren zur Herstellung von Aminen aus Alkoholen oder Carbonylverbindungen.

EP-A1-696 572, EP-A2-905 122, EP-A1-963 975, EP-A1-1 035 106, EP-A2-1 106 600 und DE-A1-10 211 101 (alle BASF AG) beschreiben Katalysatoren enthaltend Zirkoniumoxid, Kupfer- und Nickeloxid und ihre Verwendung in Verfahren zur Herstellung von Aminen aus Alkoholen bzw. Aldehyden oder Ketonen.

EP-A1-17 651 und US 4,152,353 (beide Dow Chem. Company) betrifft Katalysatoren für die Aminierung von Alkoholen, Aldehyden oder Ketonen, die Ni oder Co, Cu und (Fe, Zn oder Zr) umfassen (Anspruch 1).

Die Zirkoniumoxid-haltigen Katalysatoren des Stands der Technik sind bevorzugt durch gemeinsame Fällung von wasserlöslichen Verbindungen entsprechender Metalle einschließlich des Zirkoniums mittels Mineralbasen und anschließende Trocknung and Temperung erhältlich (Mischfällung; vgl. z.B. EP-A1-696 572, Seite 6, Zeilen 7 bis 16, und EP-A1-382 049, Seite 3, Zeilen 25 bis 35).

Man kann aber auch zunächst die Zirkoniumkomponente separat fällen und dann die übrigen Metallverbindungen (z.B. Kupfer- und Nickelverbindungen) in Gegenwart des vorher gefällten Zirkoniumoxidhydrates ausfällen (vgl. z.B. EP-A1-696 572, Seite 6, Zeilen 17 bis 23, und EP-A1-382 049, Seite 3, Zeilen 14 bis 24 und 36 bis 43).

EP-A1-1 020 424 und EP-A1-1 106 601 (beide BASF AG) beschreiben die Herstellung von Trägerkatalysatoren für die Aminierung von Acetaldehyd bzw. Aceton durch Tränkung des Trägermaterials mit entsprechenden Metallsalzlösungen. Unter den zahlreichen für die Tränkung geeigneten Trägermaterialien wird auch Zirkoniumdioxid in der monoklinen oder tetragonalen Form erwähnt.

Eine parallele deutsche Patentanmeldung (BASF AG) mit gleichem Anmeldetag betrifft ein Verfahren zur katalytischen Hydrierung einer aliphatisch ungesättigten Gruppe in einer organischen Verbindung, wobei man einen Katalysator einsetzt, bei dessen Herstellung eine Fällung von katalytisch aktiven Komponenten auf monoklines, tetragonales oder kubisches Zirkoniumdioxid erfolgte.

Eine parallele deutsche Patentanmeldung (BASF AG) mit gleichem Anmeldetag betrifft ein Verfahren zur Herstellung eines symmetrischen sekundären Amins durch Umsetzung eines primären Amins in Gegenwart von Wasserstoff und eines Katalysators, dadurch gekennzeichnet, dass man einen Katalysator einsetzt, bei dessen Herstellung eine Fällung von katalytisch aktiven Komponenten auf monoklines, tetragonales oder kubisches Zirkoniumdioxid erfolgte.

Nachteilig an den Zirkoniumdioxid-haltigen Heterogenkatalysatoren des Stands der Technik für Aminierungen von Alkoholen, Aldehyden und Ketonen ist deren erfindungsgemäß erkannte Abnahme der mechanischen Stabilität unter den Reaktionsbedingungen ihres Einsatzes, insbesondere in Gegenwart von Reaktionsmedien, die Wasser enthalten. Hierbei kann das Wasser entweder bereits im Reaktionsfeed vorhanden sein oder während der Aminierungsreaktion als Produkt entstehen. Eine Folge von mechanisch weniger stabilen Heterogenkatalysatoren ist der häufiger notwendige Katalysatorwechsel im Reaktor und damit eine erniedrigte Raum-Zeit-Ausbeute.

Der vorliegenden Erfindung lag demgemäß die Aufgabe zugrunde, den Nachteilen des Stands der Technik abzuhelfen und ein verbessertes Verfahren zur Herstellung von Aminen aus Alkoholen, Aldehyden und Ketonen bereitzustellen, wobei ein Katalysator mit verbesserten mechanischen Eigenschaften unter den Reaktionsbedingungen seines Einsatzes verwendet wird und damit die Wirtschaftlichkeit bisheriger Verfahren, insbesondere solchen, in denen Zirkoniumdioxid-haltige Katalysatoren zum Einsatz kommen, zu verbessern.

Erfindungsgemäß wurde erkannt, dass ein Grund für die verbesserungswürdige mechanische Stabilität, d.h. die mechanische Erweichung, der bekannten Zirkoniumdioxid-haltigen Katalysatoren unter Reaktionsbedingungen die Tatsache ist, dass das Zirkoniumdioxid, z.B. bei Anwendung der o.g. (Misch)Fäll-Technik, zunächst ganz oder teilweise amorph vorliegt und unter den Bedingungen der mit diesen Katalysatoren katalysierten chemischen Reaktion ganz oder teilweise eine Kristallisation, also Umwandlung der Modifikation, zu tetragonalem, monoklinem oder kubischem Zirkoniumdioxid stattfindet. Bei Aminierungsreaktionen werden als Reaktionsbedingungen zumeist eine erhöhte Temperatur (z.B. 80 - 300°C) und erhöhter Druck (z.B. 50 - 300 bar bei Flüssigphasenaminierungen bzw. 1 - 400 bar bei Gasphasenaminierungen) angewendet. Zudem sind die Reaktionsmedien wasserhaltig.

Demgemäß wurde gefunden, dass durch die Verwendung von Zirkoniumdioxid, das eine-unter den Reaktionsbedingungen der Aminierungsreaktion thermodynamisch stabile oder zumindest metastabile Modifikation aufweist, wie monoklines, tetragonales oder kubisches Zirkoniumdioxid, bei der Herstellung eines Zirkoniumdioxid-haltigen Katalysators die mechanische Stabilität der resultierenden Katalysatoren unter den Reaktionsbedingungen, insbesondere in Gegenwart von entsprechenden Reaktionsmedien, die Wasser enthalten, wesentlich erhöht wird.

Gegenstand der Erfindung ist demnach ein Verfahren zur Herstellung eines Amins durch Umsetzung eines primären oder sekundären Alkohols, Aldehyds oder Ketons mit Wasserstoff und einer Stickstoffverbindung, ausgewählt aus der Gruppe Ammoniak, primäre und sekundäre Amine, in Gegenwart eines Katalysators, welches dadurch gekennzeichnet ist, dass man einen Katalysator einsetzt, bei dessen Herstellung eine Fällung von katalytisch aktiven Komponenten auf monoklines oder tetragonales Zirkoniumdioxid erfolgte, wobei die katalytisch aktive Masse des Katalysators vor der Behandlung mit Wasserstoff
20 bis 65 Gew.-% sauerstoffhaltige Verbindungen des Zirkoniums, berechnet als ZrO₂,
1 bis 30 Gew.-% sauerstoffhaltige Verbindungen des Kupfers, berechnet als CuO,
15 bis 50 Gew.-% sauerstoffhaltige Verbindungen des Nickels, berechnet als NiO, und
15 bis 50 Gew.-% sauerstoffhaltige Verbindungen des Kobalts, berechnet als CoO,
enthält.

Bei den aufgefällten katalytisch aktiven Komponenten handelt es sich insbesondere um Metallsalze, wobei das Metall aus der Gruppe Cu, Ni und Co ausgewählt ist.

Im allgemeinen werden im erfindungsgemäßen Verfahren die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch inaktiven Begleitstoffe enthalten.

Die katalytisch aktive Masse kann nach Mahlung als Pulver oder als Splitt in das Reaktionsgefäß eingebracht oder bevorzugt, nach Mahlung, Vermischung mit Formhilfsmitteln, Formung und Temperung, als Katalysatorformkörper - beispielsweise als Tabletten, Kugeln, Ringe, Extrudate (z. B. Stränge) - in den Reaktor eingebracht werden.

Die Konzentrationsangaben (in Gew.-%) der Komponenten des Katalysators beziehen sich jeweils - falls nicht anders angegeben - auf die katalytisch aktive Masse des hergestellten Katalysators vor der Behandlung mit Wasserstoff.

Die katalytisch aktive Masse des Katalysators ist als die Summe der Massen der katalytisch aktiven Bestandteile definiert und enthält, vor der Behandlung mit Wasserstoff, im wesentlichen die katalytisch aktiven Bestandteile monoklines oder tetragonales Zirkoniumdioxid (oder Mischungen dieser Modifikationen) und Metallsalze von Cu, Ni und Co als weitere katalytisch aktive Komponenten.

Die Summe der o.g. katalytisch aktiven Bestandteile, berechnet in oxidischer Form, z.B. als ZrO₂, CuO, NiO und CoO, in der katalytisch aktive Masse vor der Behandlung mit Wasserstoff beträgt im allgemeinen 70 bis 100 Gew.-%, bevorzugt 80 bis 100 Gew.-%, besonders bevorzugt 90 bis 100 Gew.-%, insbesondere 95 bis 100 Gew.-%, ganz besonders bevorzugt >98 bis 100 Gew.-%.

Die katalytisch aktive Masse der im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen IA bis VIA und IB bis VIIB und VIII des Periodensystems, enthalten.

Beispiele für solche Elemente bzw. deren Verbindungen sind:
Übergangsmetalle, wie Mn bzw. Manganoxide, Re bzw. Rheniumoxide, Cr bzw. Chromoxide, Mo bzw. Molybdänoxide, W bzw. Wolframoxide, Ta bzw. Tantaloxide, Nb bzw. Nioboxide oder Nioboxalat, V bzw. Vanadiumoxide bzw. Vanadylpyrophosphat, Zink bzw. Zinkoxide, Silber bzw. Silberoxide, Lanthanide, wie Ce bzw. CeO₂ oder Pr bzw. Pr₂O₃, Alkalimetalloxide, wie Na₂O, Alkalimetallcarbonate, wie Na₂CO₃ und K₂CO₃, Erdalkalimetalloxide, wie SrO, Erdalkalimetallcarbonate, wie MgCO₃, CaCO₃, BaCO₃, Phosphorsäureanhydride und Boroxid (B₂O₃).

Im erfindungsgemäßen Verfahren verwendete Katalysatoren enthalten in ihrer katalytisch aktiven Masse vor der Behandlung mit Wasserstoff
20 bis 65 Gew.-%, besonders bevorzugt 22 bis 40 Gew.-%, monoklines oder tetragonales Zirkoniumdioxid (ZrO₂) (oder Mischungen dieser Modifikationen),
1 bis 30 Gew.-%, besonders bevorzugt 2 bis 25 Gew.-%, sauerstoffhaltige Verbindungen des Kupfers, berechnet als CuO, und
15 bis 50 Gew.-%, besonders bevorzugt 21 bis 45 Gew.-%, sauerstoffhaltige Verbindungen des Nickels, berechnet als NiO, wobei bevorzugt das Molverhältnis von Nickel zu Kupfer größer 1, insbesondere größer 1,2, ganz besonders 1,8 bis 8,5, ist und
15 bis 50 Gew.-%, besonders bevorzugt 21 bis 45 Gew.-%, sauerstoffhaltige Verbindungen des Kobalts, berechnet als CoO.

Die sauerstoffhaltigen Verbindungen des Kupfers, Nickels und Kobalts, jeweils berechnet als CuO, NiO und CoO, der bevorzugten Katalysatoren sind im allgemeinen insgesamt in Mengen von 15 bis 80 Gew.-%, bevorzugt 35 bis 80 Gew.-%, besonders bevorzugt 60 bis 78 Gew.-%, in der katalytisch aktiven Masse (vor der Behandlung mit Wasserstoff) enthalten, wobei besonders bevorzugt das Molverhältnis von Nickel zu Kupfer größer 1 ist.

Die erfindungsgemäß eingesetzten Katalysatoren lassen sich wie folgt herstellen.

Unter Auffällung wird eine Katalysatorherstellung verstanden, bei der ein schwer lösliches Trägermaterial in einer Flüssigkeit, meist Wasser, suspendiert wird, die Dotierungskomponenten, als leicht lösliche Verbindungen eingesetzt, in einer Flüssigkeit, meist Wasser, gelöst und dann durch Zugabe eines Fällungsmittels auf den suspendierten Träger ausgefällt werden (z.B. beschrieben in EP-A2-1 106 600, Seite 4, und A. B. Stiles, Catalyst Manufacture, Marcel Dekker, Inc., 1983, Seite 15).

Gemäß der vorliegenden Erfindung wird zur Herstellung des Katalysators als Trägermaterial Zirkoniumdioxid in einer thermodynamisch stabilen oder metastabilen Modifikation, d.h. in der monoklinen oder tetragonalen Modifikation, eingesetzt.

Die grundlegenden Eigenschaften von Zirkoniumdioxid sind in
K. Dyrek, A. Adamski, Z. Sojka, Ceramic Interfaces 2, University Press, Cambridge, 2001, S. 241 - 259,
zusammengefasst, so etwa die existierenden Modifikationen monoklin, tetragonal und kubisch und deren Herstellung.

Die Zirkoniumdioxid-Kristallstruktur kann, insbesondere bei der tetragonalen Modifikation, durch Zusätze von einem oder mehreren Metalloxiden der Nebengruppe IIIB oder der Hauptgruppe IIA des Periodensystems, insbesondere Yttriumoxid, Calciumoxid, Lanthanoxid, Magnesiumoxid oder Scandiumoxid, weiter stabilisiert sein.

Diese Stabilisierung bewirkt z.B. ganz oder teilweise eine Hemmung der Umwandlung der tetragonalen Modifikation in die thermodynamisch stabilste monokline Modifikation.

Zur Herstellung des Katalysators wird das Zirkoniumdioxid in einem Lösungsmittel, z.B. in Wasser, suspendiert. Dann werden die z.B. in Wasser gelösten Metallsalze zugegeben und anschließend durch Zugabe von z.B. Lauge in Form von im verwendeten Lösungsmittel, z.B. Wasser, schwer- oder unlöslichen, basischen Salzen gefällt.

Die bei diesen Fällungsreaktionen erhaltenen Niederschläge sind im allgemeinen chemisch uneinheitlich und enthalten in der Regel Mischungen der Oxide, Oxidhydrate, Hydroxide, Carbonate und/oder Hydrogencarbonate der eingesetzten Metalle.

Die Fällung kann z.B. bei Temperaturen von 20 - 100 °C, besonders 50 - 90 °C, insbesondere bei 60 - 80 °C, durchgeführt werden.

Alternativ kann auch die Metallsalzlösung und die Lauge gleichzeitig in einen Kessel geleitet werden, der die Zirkoniumdioxid-Trägersuspension enthält. Der Träger kann auch in der Metallsalzlösung suspendiert werden und diese dann gleichzeitig mit der Lauge in einen Fällkessel geleitet werden.

Die weitere Katalysatorherstellung wird dann nach bekannten Methoden, also etwa Filtration, Waschung, Trocknung, Calcinierung, Formgebung, Reduktion/Passivierung durchgeführt.

Die auf diese Weise hergestellten Katalysatoren enthalten vor der Reduktion/Passivierung die katalytisch aktiven Metalle in Form eines Gemisches ihrer sauerstoffhaitigen Verbindungen, d.h. insbesondere als Oxide und Mischoxide.

Die hergestellten Katalysatoren können als solche gelagert werden. Vor ihrem Einsatz als Katalysatoren zur hydrierenden Aminierung von Alkoholen, Aldehyden oder Ketonen werden sie üblicherweise durch Behandlung mit Wasserstoff vorreduziert. Sie können jedoch auch ohne Vorreduktion eingesetzt werden, wobei sie dann unter den Bedingungen der hydrierenden Aminierung durch den im Reaktor vorhandenen Wasserstoff reduziert werden. Zur Vorreduktion werden die Katalysatoren im allgemeinen zunächst bei 150 bis 200 °C über einen Zeitraum von 12 bis 20 Stunden einer Stickstoff-Wasserstoff-Atmosphäre ausgesetzt und anschließend noch bis zu ca. 24 Stunden bei 200 bis 400 °C in einer Wasserstoffatmosphäre behandelt. Bei dieser Vorreduktion wird ein Teil der in den Katalysatoren vorliegende sauerstoffhaltigen Metallverbindungen zu den entsprechenden Metallen reduziert, so dass diese gemeinsam mit den verschiedenartigen Sauerstoffverbindungen in der aktiven Form des Katalysators vorliegen.

### Amine der Formel I

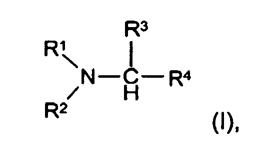

in der
- R¹, R²: Wasserstoff (H), C₁₋₂₀-Alkyl, C₃₋₁₂-Cycloalkyl, C₂₋₃₀-Alkoxyalkyl, C₃₋₃₀-Dialkylaminoalkyl, Aryl, C₇₋₂₀-Aralkyl, und C₇₋₂₀-Alkylaryl, oder gemeinsam -(CH₂)ⱼ-X-(CH₂)ₖ-,
- R³, R⁴: Wasserstoff (H), C₁₋₂₀₀-Alkyl, C₃₋₁₂Cycloalkyl, C₁₋₂₀-Hydroxyalkyl, C₁₋₂₀-Aminoalkyl, C₂₋₂₀- Hydroxyalkylaminoalkyl, C₂₋₃₀-Alkoxyalkyl, C₃₋₃₀-Dialkylaminoalkyl, C₂₋₃₀-Alkylaminoalkyl, R⁵-(OCR⁶R⁷CR⁸R⁹)ₙ-(OCR⁶R⁷), Aryl, Heteroaryl, C₇₋₂₀-Aralkyl, C₄₋₂₀-Heteroarylalkyl, C₇₋₂₀-Alkylaryl, C₄₋₂₀-Alkylheteroaryl, und Y-(CH₂)ₘ-NR⁵-(CH₂)_{q} oder gemeinsam -(CH₂)ₗ-X-(CH₂)ₘ- oder
- R² und R⁴: gemeinsam -(CH₂)ₗ-X-(CH₂)ₘ-,
- R⁵, R¹⁰: Wasserstoff (H), C₁₋₄-Alkyl, C₇₋₄₀-Alkylphenyl,
- R⁶, R⁷, R⁸, R⁹: Wasserstoff (H), Methyl oder Ethyl,
- X: CH₂, CHR⁵, Sauerstoff (O), Schwefel (S) oder NR⁵,
- Y: N(R¹⁰)₂, Hydroxy, C₂₋₂₀-Alkylaminoalkyl oder C₃₋₂₀-Dialkylaminoalkyl,
- n: eine ganze Zahl von 1 bis 30 und
- j, k, l, m, q: eine ganze Zahl von 1 bis 4,
bedeuten, sind wirtschaftlich von besonderem Interesse.

Das erfindungsgemäße Verfahren findet daher bevorzugt zur Herstellung eines Amins I Anwendung, indem man einen primären oder sekundären Alkohol der Formel II 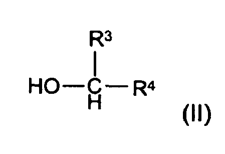 oder Aldehyd oder Keton der Formel VI bzw. VII 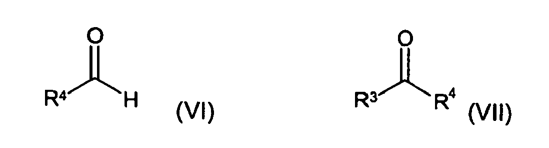 mit einer Stickstoffverbindung der Formel III 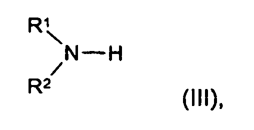 wobei R¹, R², R³ und R⁴ die oben genannten Bedeutungen haben, umsetzt.

Wie aus den Definitionen für die Reste R² und R⁴ hervorgeht, kann die Umsetzung auch intramolekular in einem entsprechenden Aminoalkohol, Aminoketon oder Aminoaldehyd erfolgen.

Zur Herstellung des Amins I wird demnach rein formal ein Wasserstoffatom der Stickstoffverbindung III durch den Rest R⁴(R³)CH- unter Freisetzung von einem Moläquivalent Wasser ersetzt.

Das erfindungsgemäße Verfahren findet auch bevorzugt Anwendung bei der Herstellung eines zyklischen Amins der Formel IV 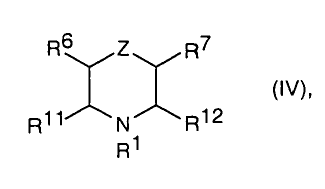 in der
- R¹¹ und R¹²: Wasserstoff (H), Alkyl, wie C₁- bis C₂₀-Alkyl, Cycloalkyl, wie C₃- bis C₁₂-Cycloalkyl, Aryl, Heteroaryl, Aralkyl, wie C₇- bis C₂₀-Aralkyl, und Alkylaryl, wie C₇- bis C₂₀-Alkylaryl,
- Z: CH₂, CHR⁵, Sauerstoff (O), NR⁵ oder NCH₂CH₂OH bedeuten und
- R¹, R⁶, R⁷: die oben genannten Bedeutungen haben,
durch Umsetzung eines Alkohols der Formel V 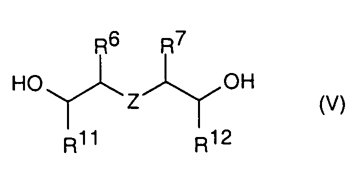 mit Ammoniak oder einem primären Amin der Formel VI

R¹―NH₂ (VI).

Die Substituenten R¹ bis R¹², die Variablen X, Y, Z und die Indizes j, k, l, m, n und q in den Verbindungen I, II, III, IV, V, VI und VII haben unabhängig voneinander folgende Bedeutungen: R¹, R², R³, R⁴, R⁵, R⁶, R⁷, R⁸, R⁹, R¹⁰, R¹¹, R¹²:
- Wasserstoff (H),
R³, R⁴:
- C₁₋₂₀₀-Alkyl, bevorzugt C₁₋₁₄-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, Cyclopentylmethyl, n-Heptyl, iso-Heptyl, Cyclohexylmethyl, n-Octyl, iso-Octyl, 2-Ethylhexyl, n-Decyl, 2-n-Propyl-n-heptyl, n-Tridecyl, 2-n-Butyl-n-nonyl und 3-n-Butyl-n-nonyl, besonders bevorzugt iso-Propyl, 2-Ethylhexyl, n-Decyl, 2-n-Propyl-n-heptyl, n-Tridecyl, 2-n-Butyl-n-nonyl und 3-n-Butyl-n-nonyl sowie bevorzugt C₄₀₋₂₀₀-Alkyl, wie Polybutyl, Polyisobutyl, Polypropyl, Polyisopropyl und Polyethyl, besonders bevorzugt Polybutyl und Polyisobutyl,
- C₁₋₂₀-Hydroxyalkyl, bevorzugt C₁₋₈-Hydroxyalkyl, besonders bevorzugt C₁₋₄-Hydroxyalkyl, wie Hydroxymethyl, 1-Hydroxyethyl, 2-Hydroxyethyl, 1-Hydroxy-n-propyl, 2-Hydroxy-n-propyl, 3-Hydroxy-n-propyl und 1-(Hydroxymethyl)ethyl,
- C₁₋₂₀-Aminoalkyl, bevorzugt C₁₋₈-Aminoalkyl, wie Aminomethyl, 2-Aminoethyl, 2-Amino-1,1-dimethylethyl, 2-Amino-n-propyl, 3-Amino-n-propyl, 4-Amino-n-butyl, 5-Amino-n-pentyl, N-(2-Aminoethyl)-2-aminoethyl und N-(2-Aminoethyl)aminomethyl,
- C₂₋₂₀-Hydroxyalkylaminoalkyl, bevorzugt C₃₋₈₋-Hydroxyalkylaminoalkyl, wie (2-Hydroxyethylamino)methyl, 2-(2-Hydroxyethylamino)ethyl und 3-(2-Hydroxyethylamino)propyl,
- R⁵-(OCR⁶R⁷CR^{B}R⁹)ₙ-(OCR⁶R⁷), bevorzugt R⁵-(OCHR⁷CHR⁹)ₙ-(OCR⁶R⁷) besonders bevorzugt R⁵-(OCH₂CHR⁹)ₙ-(OCR⁶R⁷),
- C₂₋₃₀-Alkylaminoalkyl, bevorzugt C₂₋₂₀-Alkylaminoalkyl, besonders bevorzugt C₂₋₈-Alkylaminoalkyl, wie Methylaminomethyl, 2-Methylaminoethyl, Ethylaminomethyl, 2-Ethylaminoethyl und 2-(iso-Propylamino)ethyl, (R⁵)HN-(CH₂)_{q},
- Y-(CH₂)ₘ-NR⁵-(CH₂)_{q},
- C₄₋₂₀-Heteroarylalkyl, wie Pyrid-2-yl-methyl, Furan-2-yl-methyl, Pyrrol-3-yl-methyl und Imidazol-2-yl-methyl,
- C₄₋₂₀-Alkylheteroaryl, wie 2-Methyl-3-pyridinyl, 4,5-Dimethyl-imidazol-2-yl, 3-Methyl-2-furanyl und 5-Methyl-2-pyrazinyl,
- Heteroaryl, wie 2-Pyridinyl, 3-Pyridinyl, 4-Pyridinyl, Pyrazinyl, Pyrrol-3-yl, Imidazol-2-yl, 2-Furanyl und 3-Furanyl,
R¹, R², R³, R⁴:
- C₃₋₁₂-Cycloalkyl, bevorzugt C₃₋₈-Cycloalkyl, wie Cyclopropyl, Cyclobutyl, Cyclopentyl, Cyclohexyl, Cycloheptyl und Cyclooctyl, besonders bevorzugt Cyclopentyl und Cyclohexyl,
- C₂₋₃₀-Alkoxyalkyl, bevorzugt C₂₋₂₀-Alkoxyalkyl, besonders bevorzugt C₂₋₈-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxy-ethyl und 2-Methoxyethyl, besonders bevorzugt C₂₋₄-Alkoxyalkyl,
- C₃₋₃₀-Dialkylaminoalkyl, bevorzugt C₃₋₂₀-Dialkylaminoalkyl, besonders bevorzugt C₃₋₁₀-Dialkylaminoalkyl, wie N,N-Dimethylaminomethyl, (N,N-Dibutylamino)methyl, 2-(N,N-Dimethylamino)ethyl, 2-(N,N-Diethylamino)ethyl, 2-(N,N-Dibutylamino)ethyl, 2-(N,N-Di-n-propylamino)ethyl und 2-(N,N-Di-isopropylamino)ethyl, 3--(N,N-Dimethylamino)propyl, (R⁵)₂N-(CH₂)_{q},
- Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- C₇₋₂₀-Alkylaryl, bevorzugt C₇₋₁₂-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
- C₇₋₂₀-Aralkyl, bevorzugt C₇₋₁₂-Phenylalkyl, wie Benzyl, p-Methoxybenzyl, 3,4-Dimethoxybenzyl, 1-Phenethyl, 2-Phenethyl, 1-Phenyl-propyl, 2-Phenylpropyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- R³ und R⁴ oder R² und R⁴ gemeinsam eine -(CH₂)ₗ-X-(CH₂)ₘ- Gruppe, wie - (CH₂)₃-, -(CH₂)₄-, -(CH₂)₅-, -(CH₂)₆-, -(CH₂)₇-, -(CH₂)-O-(CH₂)₂-, -(CH₂)-NR⁵-(CH₂)₂-, -(CH₂)-CHR⁵-(CH₂)₂-, -(CH₂)₂-O-(CH₂)₂-, -(CH₂)₂-NR⁵-(CH₂)₂-, -(CH₂)₂-CHR⁵-(CH₂)₂-, -CH₂-O-(CH₂)₃-, -CH₂-NR⁵-(CH₂)₃-, -CH₂CHR⁵-(CH₂)₃-,
R¹, R²:
- C₁₋₂₀-Alkyl, bevorzugt C₁₋₈-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, 2-Ethylhexyl, besonders bevorzugt C₁₋₄-Alkyl, oder
- R¹ und R² gemeinsam eine -(CH₂)ⱼ-X-(CH₂)ₖ- Gruppe, wie -(CH₂)₃-, -(CH₂)₄-, -(CH₂)₅-, -(CH₂)₆-, -(CH₂)₇-, -(CH₂)-O-(CH₂)₂-, -(CH₂)-NR⁵-(CH₂)₂-, -(CH₂)-CHR⁵-(CH₂)₂-, -(CH₂)₂-O-(CH₂)₂-, -(CH₂)₂-NR⁵-(CH₂)₂-, -(CH₂)₂-CHR⁵-(CH₂)₂-, -CH₂-O-(CH₂)₃-, -CH₂-NR⁵-(CH₂)₃-, -CH₂-CHR⁵-(CH₂)₃-,
R⁵, R¹⁰:
- C₁₋₄-Alkyl, wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,
- C₇₋₄₀-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2-, 3-, 4-Nonylphenyl, 2-, 3-, 4-Decylphenyl, 2,3-, 2,4-, 2,5-, 3,4-, 3,5-Dinonylphenyl, 2,3-, 2,4-, 2,5-, 3,4- und 3,5-Didecylphenyl,
R⁶, R⁷, R⁸, R⁹_{:}
- Methyl oder Ethyl, bevorzugt Methyl,
R¹¹ R¹²:
- C₁- bis C₂₀-Alkyl, C₃- bis C₁₂-Cycloalkyl, Aryl, Heteroaryl, C₇- bis C₂₀-Aralkyl, und C₇- bis C₂₀-Alkylaryl, jeweils wie oben definiert,
X:
- CH₂, CHR⁵, Sauerstoff (O), Schwefel (S) oder NR⁵, bevorzugt CH₂ und O,
Y:
- N(R¹⁰)₂, bevorzugt NH₂ und N(CH₃)₂,
- Hydroxy (OH),
- C₂₋₂₀-Alkylaminoalkyl, bevorzugt C₂₋₁₆-Alkylaminoalkyl, wie Methylaminomethyl, 2-Methylaminoethyl, Ethylaminomethyl, 2-Ethylaminoethyl und 2-(isoPropylamino)ethyl,
- C₃₋₂₀-Dialkylaminoalkyl, bevorzugt C₃₋₁₆-Dialkylaminoalkyl, wie Dimethylaminomethyl, 2-Dimethylaminoethyl, 2-Diethylaminoethyl, 2-(Di-n-propylamino)ethyl und 2-(Di-iso-propylamino)ethyl,
Z:
- CH₂, CHR⁵, O, NR⁵ oder NCH₂CH₂OH,
j, l:
- eine ganze Zahl von 1 bis 4 (1, 2, 3 oder 4), bevorzugt 2 und 3, besonders bevorzugt 2,
k, m, q:
- eine ganze Zahl von 1 bis 4 (1, 2, 3 oder 4), bevorzugt 2, 3 und 4, besonders bevorzugt 2 und 3,
n:
- eine ganze Zahl von 1 bis 30, bevorzugt eine ganze Zahl von 1 bis 8 (1, 2, 3, 4, 5, 6, 7 oder 8), besonders bevorzugt eine ganze Zahl von 1 bis 6.

Als Alkohole eignen sich praktisch alle primären und sekundären Alkohole mit aliphatischer OH-Funktion. Die Alkohole können geradkettig, verzweigt oder zyklisch sein. Sekundäre Alkohole werden ebenso aminiert wie primäre Alkohole. Hinsichtlich der Kohlenstoffzahl der aminierbaren Alkohole gibt es praktisch keine Beschränkungen. Die Alkohole können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige Alkohole aminiert werden, so hat man es über die Steuerung der Reaktionsbedingungen in der Hand, bevorzugt Aminoalkohole, zyklische Amine oder mehrfach aminierte Produkte zu erhalten.

Die Aminierung von 1,4-Diolen führt je nach Wahl der Reaktionsbedingungen zu 1-Amino-4-hydroxy-, 1,4-Diamino-Verbindungen oder zu fünfgliedrigen Ringen mit einem Stickstoffatom (Pyrrolidinen).

Die Aminierung von 1,6-Diolen führt je nach Wahl der Reaktionsbedingungen zu 1-Amino-6-hydroxy-, 1,6-Diamino-Verbindungen oder zu siebengliedrigen Ringen mit einem Stickstoffatom (Hexamethyleniminen).

Die Aminierung von 1,5-Diolen führt je nach Wahl der Reaktionsbedingungen zu 1-Amino-5-hydroxy-, 1,5-Diamino-Verbindungen oder zu sechsgliedrigen Ringen mit einem Stickstoffatom (Piperidinen). Aus Diglykol kann demnach durch Aminierung mit NH₃ Monoaminodiglykol (= ADG = H₂N-CH₂CH₂-O-CH₂CH₂-OH), Diaminodiglykol oder besonders bevorzugt Morpholin erhalten werden. Aus Diethanolamin wird entsprechend besonders bevorzugt Piperazin erhalten. Aus Triethanolamin kann N-(2-Hydroxyethyl)-piperazin erhalten werden.

Bevorzugt werden beispielsweise die folgenden Alkohole aminiert:
Methanol, Ethanol, n-Propanol, Isopropanol, n-Butanol, iso-Butanol, tert.-Butanol, n-Pentanol, n-Hexanol, 2-Ethylhexanol, Tridecanol, Stearylalkohol, Palmitylalkohol, Cyclobutanol, Cyclopentanol, Cyclohexanol, Benzylalkohol, 2-Phenyl-ethanol, 2-(p-Methoxyphenyl)ethanol, 2-(3,4-Dimethoxyphenyl)ethanol, 1-Phenyl-3-butanol, Ethanolamin, n-Propanolamin, Isopropanolamin, 2-Amino-1-propanol, 1-Methoxy-2-propanol, 3-Amino-2,2-dimethyl-1-propanol, n-Pentanolamin (1-Amino-5-pentanol), n-Hexanolamin (1-Amino-6-hexanol), Ethanolamin, Diethanolamin, Triethanolamin, N-Alkyldiethanolamine, Diisopropanolamin, 3-(2-Hydroxyethylamino)propan-1-ol, 2-(N,N-Dimethylamino)ethanol, 2-(N,N-Diethylamino)ethanol, 2-(N,N-Di-n-propylamino)ethanol, 2-(N,N-Di-iso-propylamino)ethanol, 2-(N,N-Di-n-butylamino)ethanol, 2-(N,N-Di-iso-butylamino)ethanol, 2-(N,N-Di-sec.-butylamino)ethanol, 2-(N,N-Di-tert.butylamino)ethanol, 3-(N,N-Dimethylamino)propanol, 3-(N,N-Diethylamino)propanol, 3-(N,N-Di-n-propylamino)propanol, 3-(N,N-Di-iso-propylamino)propanol, 3-(N,N-Di-nbutylamino)propanol, 3-(N,N-Di-iso-butylamino)propanol, 3-(N,N-Di-sec.-butylamino)propanol, 3-(N,N-Di-tert.-butylamino)propanol, 1-Dimethylamino-pentanol-4, 1-Diethylamino-pentanol-4, Ethylenglykol, 1,2-Propylenglykol, 1,3-Propylenglykol, Diglykol, 1,4-Butandiol, 1,5-Pentandiol, 1,6-Hexandiol, 2,2-Bis[4-hydroxycyclohexyl]propan, Methoxyethanol, Propoxyethanol, Butoxyethanol, Polyisobutylalkohole, Polypropylalkohole, Polyethylenglykolether, Polypropylenglykolether und Polybutylenglykolether. Die letztgenannten Polyalkylenglykolether werden bei der erfindungsgemäßen Umsetzung durch Umwandlung ihrer freien Hydroxylgruppen zu den entsprechenden Aminen umgewandelt.

Besonders bevorzugte Alkohole sind Methanol, Ethanol, n-Propanol, i-Propanol, n-Butanol, sek.-Butanol, 2-Ethylhexanol, Fettalkohole, Ethylenglykol, Diethylenglykol, 2-(2-Dimethylamino-ethoxy)ethanol, N-Methyldiethanolamin und 2-(2-Dimethylaminoethoxy)ethanol.

Als im erfindungsgemäßen Verfahren einsetzbare Ketone eignen sich praktisch alle aliphatischen und aromatischen Ketone. Die aliphatischen Ketone können geradkettig, verzweigt oder zyklisch sein, die Ketone können Heteroatome enthalten. Hinsichtlich der Kohlenstoffzahl der aminierbaren Ketone gibt es praktisch keine Beschränkungen. Die Ketone können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige Ketone aminiert werden, so hat man es über die Steuerung der Reaktionsbedingungen in der Hand, Aminoketone, Aminoalkohole, cyclische Amine oder mehrfach aminierte Produkte zu erhalten.

Bevorzugt werden beispielsweise die folgenden Ketone aminierend hydriert:
Aceton, Ethylmethylketon, Methylvinylketon, Isobutylmethylketon, 3-Methylbutan-2-on, Diethylketon, Tetralon, Acetophenon, p-Methyl-acetophenon, p-Methoxy-acetophenon, m-Methoxy-acetophenon, 1-Acetyl-naphthalin, 2-Acetyl-naphthalin, 1-Phenyl-3-butanon, Cyclobutanon, Cyclopentanon, Cyclopentenon, Cyclohexanon, Cyclohexenon, 2,6-Dimethylcyclohexanon, Cycloheptanon, Cyclododecanon, Acetylaceton, Methylglyoxal und Benzophenon.

Als im erfindungsgemäßen Verfahren einsetzbare Aldehyde eignen sich praktisch alle aliphatischen und aromatischen Aldehyde. Die aliphatischen Aldehyde können geradkettig, verzweigt oder zyklisch sein, die Aldehyde können Heteroatome enthalten. Hinsichtlich der Kohlenstoffzahl der aminierbaren Aldehyde gibt es praktisch keine Beschränkungen. Die Aldehyde können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige Aldehyde oder Ketoaldehyde aminiert werden, so hat man es über die Steuerung der Reaktionsbedingungen in der Hand, Aminoalkohole, cyclische Amine oder mehrfach aminierte Produkte zu erhalten.

Bevorzugt werden beispielsweise die folgenden Aldehyde aminierend hydriert:
Formaldehyd, Acetaldehyd, Propionaldehyd, n-Butyraldehyd, Isobutyraldehyd, Pivalinaldehyd, n-Pentanal, n-Hexanal, 2-Ethylhexanal, 2-Methylpentanal, 3-Methylpentanal, 4-Methylpentanal, Glyoxal, Benzaldehyd, p-Methoxybenzaldehyd, p-Methylbenzaldehyd, Phenylacetaldehyd, (p-Methoxy-phenyl)acetaldehyd, (3,4-Dimethoxyphenyl)acetaldehyd, 4-Formyltetrahydropyran, 3- Formyltetrahydrofuran, 5-Formylvaleronitril, Citronellal, Acrolein, Methacrolein, Ethylacrolein, Citral, Crotonaldehyd, 3-Methoxypropionaldehyd, 3-Aminopropionaldehyd, Hydroxypivalinaldehyd, Dimethylolpropionaldehyd, Dimethylolbutyraldehyd, Furfural, Glyoxal, Glutaraldehyd sowie hydroformylierte Oligomere und Polymere, wie z. B. hydroformyliertes Polyisobuten (Polyisobutenaldehyd) oder durch Metathese von 1-Penten und Cyclopenten erhaltenes und hydroformyliertes Oligomer.

Als Aminierungsmittel bei der hydrierenden Aminierung von Alkoholen, Aldehyden oder Ketonen in Gegenwart von Wasserstoff können sowohl Ammoniak als auch primäre oder sekundäre, aliphatische oder cycloaliphatische oder aromatische Amine eingesetzt werden.

Bei Verwendung von Ammoniak als Aminierungsmittel wird die alkoholische Hydroxylgruppe bzw. die Aldehydgruppe bzw. die Ketogruppe zunächst in die primäre Aminogruppen (-NH₂) umgewandelt. Das so gebildete primäre Amin können mit weiterem Alkohol bzw. Aldehyd bzw. Keton zu dem entsprechenden sekundären Amin und diese wiederum mit weiterem Alkohol bzw. Aldehyd bzw. Keton zu dem entsprechenden, vorzugsweise symmetrischen, tertiären Amin reagieren. Je nach Zusammensetzung des Reaktionsansatzes oder des Eduktstroms (bei kontinuierlicher Fahrweise) und je nach den angewandten Reaktionsbedingungen - Druck, Temperatur, Reaktionszeit (Katalysatorbelastung) - lassen sich auf diese Weise je nach Wunsch bevorzugt primäre, sekundäre oder tertiäre Amine darstellen.

Aus mehrwertigen Alkoholen bzw. Di- oder Oligoaldehyden bzw. Di- oder Oligoketonen bzw. Ketoaldehyden lassen sich auf diese Weise durch intramolekulare hydrierende Aminierung zyklische Amine wie z.B. Pyrrolidine, Piperidine, Hexamethylenimine, Piperazine und Morpholine herstellen.

Ebenso wie Ammoniak lassen sich primäre oder sekundäre Amine als Aminierungsmittel verwenden.

Bevorzugt werden diese Aminierungsmittel zur Herstellung unsymmetrisch substituierter Di- oder Trialkylamine, wie Ethyldiisopropylamin und Ethyldicyclohexylamin verwendet. Beispielsweise werden die folgenden Mono- und Dialkylamine als Aminierungsmittel verwendet: Methylamin, Dimethylamin, Ethylamin, Diethylamin, n-Propylamin, Di-n-propyl-amin, iso-Propylamin, Di-isopropyl-amin, Isopropylethylamin, n-Butylamin, Di-n-Butylamin, s-Butylamin, Di-s-Butylamin, iso-Butylamin, n-Pentylamin, s-Pentylamin, iso-Pentylamin, n-Hexylamin, s-Hexylamin, iso-Hexylamin, Cyclohexylamin, Anilin, Toluidin, Piperidin, Morpholin und Pyrrolidin.

Mit dem erfindungsgemäßen Verfahren besonders bevorzugt hergestellte Amine sind zum Beispiel Morpholin (aus Aminodiglykol), 6-Dimethylaminohexanol-1 (aus Hexandiol und Dimethylamin (DMA)), Triethylamin (aus Ethanol und Diethylamin (DEA)), Dimethylethylamin (aus Ethanol und DMA), N-Methylmorpholin (aus Diethylenglykol und Monomethylamin (MMA)), N-Methylpiperidin (Pentandiol und MMA), N-Methylpiperazin (aus Diethanolamin und MMA), N,N'-Dimethylpiperazin (aus N-Methyldiethanolamin und MMA), Ethylendiamin (EDA) und Diethylentriamin (DETA) aus Monoethanolamin (MEOA), 2-Ethylhexylamin und Bis(2-Ethylhexyl)amin (aus 2-Ethylhexanol und NH₃), Tridecylamin und Bis(Tridecyl)amin (aus Tridecanol und NH₃), n-Octylamin (aus n-Octanol und NH₃), 1,2-Propylendiamin (aus 2-Hydroxy-propylamin und NH₃), 1-Diethylamino-4-aminopentan (aus 1-Diethylamino-4-hydroxypentan und NH₃), Dimethylcyclohexylamin (aus Cyclohexanon und DMA), Polyisobutenamin (aus Pib-Oxo und NH₃), Propylamine (wie Mono-/Dipropylamin, Dimethylpropylamin) (aus Propionaldehyd oder n-Propanol und NH₃ bzw. DMA).

Das Aminierungsmittel kann bezüglich der zu aminierenden alkoholischen Hydroxylgruppe bzw. Aldehydgruppe bzw. Ketogruppe in stöchiometrischen, unter- oder überstöchiometrischen Mengen eingesetzt werden.

Bevorzugt wird im Falle der Aminierung von Alkoholen, Aldehyden oder Ketonen mit primären oder sekundären Aminen das Amin in ca. stöchiometrischer Menge oder geringfügig überstöchiometrischer Menge pro Mol zu aminierender alkoholischer Hydroxylgruppe, Aldehydgruppe oder Ketogruppe eingesetzt.

Speziell Ammoniak wird im allgemeinen mit einem 1,5 bis 250-fachen, bevorzugt 2 bis 100-fachen, insbesondere 2 bis 10-fachen molaren Überschuss pro Mol umzusetzender alkoholischer Hydroxylgruppe, Aldehydgruppe oder Ketogruppe eingesetzt.
Höhere Überschüsse sowohl an Ammoniak als auch an primären oder sekundären Aminen sind möglich.

Das erfindungsgemäße Verfahren lässt sich diskontinuierlich oder bevorzugt kontinuierlich wie folgt durchführen, wobei der Katalysator bevorzugt als Festbett im Reaktor angeordnet ist.
Die Ausführungsform als Wirbelbettreaktion mit in auf- und abwirbelnder Bewegung befindlichem Katalysatormaterial ist jedoch ebenfalls möglich.

Die Aminierung der primären oder sekundären Alkoholgruppen, Aldehydgruppen oder Ketogruppen des Edukts kann in der Flüssigphase oder in der Gasphase durchgeführt werden. Bevorzugt ist das Festbettverfahren in der Gasphase.

Beim Arbeiten in der Flüssigphase leitet man die Edukte (Alkohol, Aldehyd oder Keton plus Ammoniak oder Amin) simultan in flüssiger Phase bei Drücken von im allgemeinen 5 bis 30 MPa (50-300 bar), bevorzugt 5 bis 25 MPa, besonders bevorzugt 15 bis 25 MPa, und Temperaturen von im allgemeinen 80 bis 300°C, bevorzugt 120 bis 270°C, besonders bevorzugt 130 bis 250°C, insbesondere 170 bis 230°C, inklusive Wasserstoff über den Katalysator, der sich üblicherweise in einem bevorzugt von außen beheizten Festbettreaktor befindet. Es ist dabei sowohl eine Rieselfahrweise als auch eine Sumpffahrweise möglich. Die Katalysatorbelastung liegt im allgemeinen im Bereich von 0,05 bis 5, bevorzugt 0,1 bis 2, besonders bevorzugt 0,2 bis 0,6, kg Alkohol, Aldehyd oder Keton pro Liter Katalysator (Schüttvolumen) und Stunde. Gegebenenfalls kann eine Verdünnung der Edukte mit einem geeigneten Lösungsmittel, wie Tetrahydrofuran, Dioxan, N-Methylpyrrolidon oder Ethylenglykoldimethylether, erfolgen. Es ist zweckmäßig, die Reaktanden bereits vor der Zuführung in das Reaktionsgefäß zu erwärmen, und zwar bevorzugt auf die Reaktionstemperatur.

Beim Arbeiten in der Gasphase werden die gasförmigen Edukte (Alkohol, Aldehyd oder Keton plus Ammoniak oder Amin) in einem zur Verdampfung ausreichend groß gewählten Gasstrom, bevorzugt Wasserstoff, bei Drücken von im allgemeinen 0,1 bis 40 MPa (1 bis 400 bar), bevorzugt 0,1 bis 10 MPa, besonders bevorzugt 0,1 bis 5 MPa, in Gegenwart von Wasserstoff über den Katalysator geleitet. Die Temperaturen für die Aminierung von Alkoholen betragen im allgemeinen 80 bis 300°C, bevorzugt 120 bis 270°C, besonders bevorzugt 160 bis 250°C. Die Reaktionstemperaturen bei der hydrierenden Aminierung von Aldehyden und Ketonen betragen im allgemeinen bei 80 bis 300°C, bevorzugt bei 100 bis 250°C. Es ist dabei sowohl eine Anströmung des Katalysatorfestbetts von oben als auch von unten möglich. Den erforderlichen Gasstrom erhält man bevorzugt durch eine Kreisgasfahrweise.
Die Katalysatorbelastung liegt im allgemeinen im Bereich von 0,01 bis 2, bevorzugt 0,05 bis 0,5, kg Alkohol, Aldehyd oder Keton pro Liter Katalysator (Schüttvolumen) und Stunde.

Der Wasserstoff wird der Reaktion im allgemeinen in einer Menge von 5 bis 400 l, bevorzugt in einer Menge von 50 bis 200 l pro Mol Alkohol-, Aldehyd- oder Ketonkomponente zugeführt, wobei die Literangaben jeweils auf Normalbedingungen umgerechnet wurden (S.T.P.).
Die Aminierung von Aldehyden bzw. Ketonen unterscheidet sich in der Durchführung von der Aminierung von Alkoholen dadurch, dass bei der Aminierung von Aldehyden und Ketonen mindestens stöchiometrische Mengen an Wasserstoff vorhanden sein müssen.

Sowohl beim Arbeiten in der Flüssigphase als auch beim Arbeiten in der Gasphase ist die Anwendung höherer Temperaturen und höherer Gesamtdrücke möglich. Der Druck im Reaktionsgefäß, welcher sich aus der Summe der Partialdrücke des Aminierungsmittels, des Alkohols, Aldehyds bzw. Ketons und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck erhöht.

Sowohl beim kontinuierlichen Arbeiten in der Flüssigphase als auch beim kontinuierlichen Arbeiten in der Gasphase kann das überschüssige Aminierungsmittel zusammen mit dem Wasserstoff im Kreis geführt werden.

Ist der Katalysator als Festbett angeordnet, kann es für die Selektivität der Reaktion vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Das im Zuge der Umsetzung gebildete Reaktionswasser (jeweils ein Mol pro Mol umgesetzte Alkoholgruppe, Aldehydgruppe bzw. Ketogruppe) wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z. B. destillativ.

Aus dem Reaktionsaustrag werden, nachdem dieser zweckmäßigerweise entspannt worden ist, das überschüssige Aminierungsmittel und der Wasserstoff entfernt und die erhaltenen Aminierungsprodukte durch Destillation bzw. Rektifikation, Flüssigextraktion oder Kristallisation gereinigt. Das überschüssige Aminierungsmittel und der Wasserstoff werden vorteilhaft wieder in die Reaktionszone zurückgeführt. Das gleiche gilt für die eventuell nicht vollständig umgesetzte Alkohol-, Aldehyd- bzw. Ketonkomponente.

Die unter Anwendung des erfindungsgemäßen Verfahrens hergestellten Amine eignen sich u. a. als Zwischenprodukte bei der Herstellung von Kraftstoffadditiven (US-A-3,275,554; DE-A-21 25 039 und DE-A-36 11 230), Tensiden, Arznei- und Pflanzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren.

### Beispiele

### Herstellung von Katalysatoren

### Katalysator A (erfindungsgemäß):

Katalysator A wurde durch Auffällung der Komponenten Cu, Co, Ni auf monoklines Zirkoniumdioxid, das im Fällkessel vorgelegt wurde, wie folgt hergestellt:

In einem Rührkessel aus Glas wurde eine Suspension aus 155 g monoklinem Zirkoniumdioxid-Pulver (BET = 105 m² g⁻¹) in 2 l Wasser vorgelegt und unter Rühren auf 70°C erhitzt. Innerhalb von 30 Min. wurde dann eine Lösung aus 190,1 g Cu(NO₃)₂ x 2,5 H₂O, 561,9 g Ni(NO₃)₂ x 6 H₂O und 543,7 g Co(NO₃)₂ x 6 H₂O in 2,8 l Wasser zugetropft. Durch gleichzeitiges Zutropfen einer 20 %-igen Sodalösung (700 g Na₂CO₃ in 2,8 I Wasser) wurde der pH bei konstant 6,0 gehalten. Nach der Zugabe der Lösungen wurde für 1 h bei 70°C nachgerührt und schließlich durch Zugabe von Sodalösung der pH-Wert auf 7,4 erhöht. Die Suspension wurde auf eine Nutsche gepumpt und mit 100 I Wasser gewaschen. Der Filterkuchen wurde für 16 h bei 200°C im Trockenschrank getrocknet, anschließend auf Korngrößen < 1,6 mm zerkleinert und für 2 h bei 400°C in einem Drehrohrofen im Luftstrom (150 l/h) calciniert.

Das so erhaltene Katalysatorpulver hatte die Zusammensetzung:
28 Gew.-% Ni, berechnet als NiO,
28 Gew.-% Co, berechnet als CoO,
11 Gew.-% Cu, berechnet als CuO, und
33 Gew.-% Zr, berechnet als ZrO₂.

Das Pulver wurde mit 3 Gew.-% Graphit versetzt, kompaktiert, wieder auf < 1,6 mm zerkleinert, und schließlich zu 4,75 x 3 mm-Tabletten gepresst. Die Tabletten wurden für 2 h bei 400°C im Muffelofen an Luft nachcalciniert. In einer Reduktionskolonne wurden die Tabletten dann in einem Wasserstoff/Stickstoffstrom reduziert, zunächst 6 h bei 150°C, dann 6 h bei 280°C. Nach Abkühlung auf Raumtemperatur wurden die Tabletten dann in einem verdünnten Luftstrom passiviert.

### Katalysator B (Vergleichskatalysator):

Der Vergleichskatalysator wurde wie der erfindungsgemäße Katalysator A hergestellt, mit dem Unterschied, dass kein monoklines Zirkoniumdioxid vorgelegt wurde. Anstelle dessen wurden zu der Cu-, Co- und Ni-Nitrathaltigen Metallsalzlösung 775 g einer Zirkoniumacetatlösung, enthaltend 20 Gew.-% ber. ZrO₂, (bezogen auf das Gewicht der Zirkoniumacetatlösung) zugegeben (Mischfällung). Die weitere Herstellung erfolgte analog Katalysator A. Das analog erhaltene Katalysatorpulver hatte die gleiche Zusammensetzung wie beim Katalysator A beschrieben.

### Beispiel 1:

Zur Testung der mechanischen Stabilität der Katalysatoren A und B unter den Reaktionsbedingungen der hydrierenden Aminierung von hydroformylierten Polyisobuten (PIB-Oxo; Molmasse: 1000) zum primären Amin PIBA nach dem Reaktionsschema 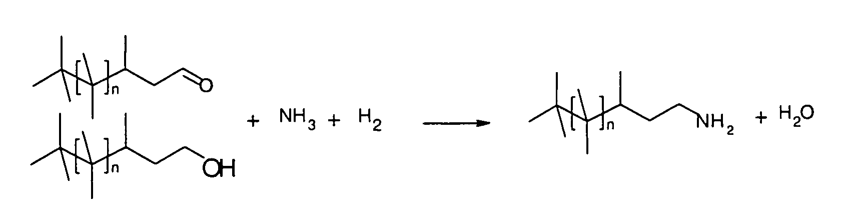 wurden die Katalysatoren in einem Autoklaven einem sog. Kochtest (Autoklaventest) unterzogen, bei dem die Reaktionsbedingungen wie folgt nachgestellt wurden:
20 g Katalysator wurden in einen Drahtkorb in einem Rührautoklaven gegeben. Dazu wurden 150 ml einer PIBA/Mihagol (50/50) Lösung gegeben, so dass die Katalysatortabletten gut mit Flüssigkeit überdeckt waren. Der Autoklav wurde verschlossen, mit Stickstoff gespült, der Rührer mit 700 U/min betrieben, 50 bar H₂ aufgepresst und auf 200 °C aufgeheizt. Der Druck wurde dann durch Zupressen von H₂ auf 200 bar eingestellt. Unter diesen Bedingungen wurde der Katalysator für unterschiedliche Zeiträume behandelt. Anschließend wurde abgekühlt, vorsichtig entspannt und die mechanische Stabilität des Katalysators durch Messung der Seitendruckfestigkeit (SDF) bestimmt.

Hierzu wurde die Katalysatortablette zwischen zwei parallelen Platten auf der Mantelseite mit zunehmender Kraft belastet, bis Bruch eintrat. Die beim Bruch registrierte Kraft ist die Seitendruckfestigkeit. Die Bestimmung erfolgte auf einem Prüfgerät der Firma Zwick, Ulm, mit festsitzendem Drehteller und frei beweglichem, vertikalem Stempel, der den Formkörper gegen den festsitzenden Drehteller drückte. Der frei bewegliche Stempel war mit einer Druckmessdose zur Aufnahme der Kraft verbunden. Das Gerät wurde durch einen Computer gesteuert, welcher die Meßwerte registrierte und auswertete. Aus einer gut durchmischten Katalysatorprobe wurden 25 einwandfreie (d.h. rissefrei und ohne abgestoßenen Kanten) Tabletten entnommen, deren Seitendruckfestigkeit ermittelt und anschließend gemittelt.

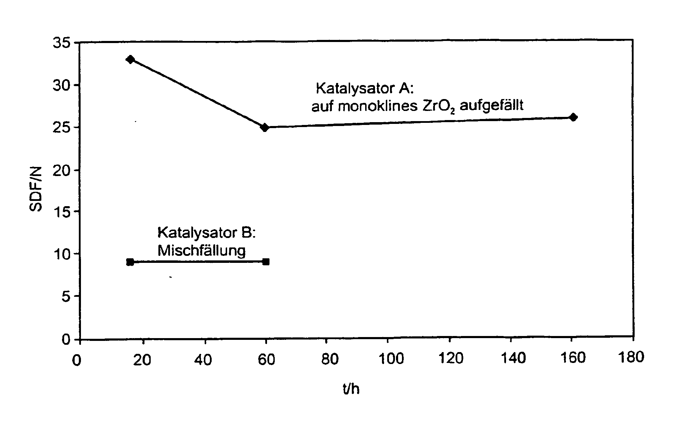

In der beigefügten Abbildung 1 sind die Seitendruckfestigkeiten (Einheit: Newton, N) der beiden Katalysatoren A und B über die Dauer des Autoklaventests (in Stunden) aufgetragen:

Bei dem durch Mischfällung hergestellten Kontakt B zeigte das Röntgendiffraktogramm nach erfolgtem Kochtest, dass sich die zunächst röntgenamorphe ZrO₂-Phase in tetragonales und monoklines ZrO₂ umgewandelt hatte. Bei Katalysator A wurde keine Umkristallisation (= Änderung der Modifikation) festgestellt.

## Patentansprüche

1. Verfahren zur Herstellung eines Amins durch Umsetzung eines primären oder sekundären Alkohols, Aldehyds oder Ketons mit Wasserstoff und einer Stickstoffverbindung, ausgewählt aus der Gruppe Ammoniak, primäre und sekundäre Amine, in Gegenwart eines Katalysators, **dadurch gekennzeichnet, dass** man einen Katalysator einsetzt, bei dessen Herstellung eine Fällung von katalytisch aktiven Komponenten auf monoklines oder tetragonales Zirkoniumdioxid erfolgte, wobei die katalytische aktive Masse des Katalysators vor der Behandlung mit Wasserstoff
20 bis 65 Gew.-% sauerstoffhaltige Verbindungen des Zirkoniums, berechnet als ZrO₂,
1 bis 30 Gew.-% sauerstoffhaltige Verbindungen des Kupfers, berechnet als CuO,
15 bis 50 Gew.-% sauerstoffhaltige Verbindungen des Nickels, berechnet als NiO, und
15 bis 50 Gew.-% sauerstoffhaltige Verbindungen des Kobalts, berechnet als CoO, enthält.

2. Verfahren nach dem vorhergehenden Anspruch, **dadurch gekennzeichnet, dass** es sich bei den Metallsalzen um in Wasser schwer- oder unlösliche, basische Salze handelt.

3. Verfahren nach einem der beiden vorhergehenden Ansprüche, **dadurch gekennzeichnet, dass** es sich bei den Salzen um Oxide, Oxidhydrate, Hydroxide, Carbonate und/oder Hydrogencarbonate handelt.

4. Verfahren nach Anspruch 1, **dadurch gekennzeichnet, dass** das Molverhältnis von Nickel zu Kupfer größer 1 ist.

5. Verfahren nach einem der vorhergehenden Ansprüche, **dadurch gekennzeichnet, dass** das monokline oder tetragonale Zirkoniumdioxid ein oder mehrere Metalloxide der Nebengruppe IIIB oder der Hauptgruppe IIA des Periodensystems enthält.

6. Verfahren nach einem der vorhergehenden Ansprüche, **dadurch gekennzeichnet, dass** man die Umsetzung bei Temperaturen von 80 bis 300°C durchführt.

7. Verfahren nach einem der vorhergehenden Ansprüche, **dadurch gekennzeichnet, dass** man die Umsetzung in der Flüssigphase bei Drücken von 5 bis 30 MPa oder in der Gasphase bei Drücken von 0,1 bis 40 MPa durchführt.

8. Verfahren nach einem der vorhergehenden Ansprüche zur Herstellung eines Amins der Formel I 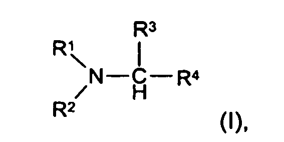 in der
R¹, R² Wasserstoff (H), C₁₋₂₀-Alkyl, C₃₋₁₂-Cycloalkyl, C₂₋₃₀-Alkoxyalkyl, C₃₋₃₀-Dialkylaminoalkyl, Aryl, C₇₋₂₀-Aralkyl, C₇₋₂₀-Alkylaryl, oder gemeinsam -(CH₂)ⱼ-X-(CH₂)ₖ-,
R³, R⁴ Wasserstoff (H), C₁₋₂₀₀-Alkyl, C₃₋₁₂-Cycloalkyl, C₁₋₂₀-Hydroxyalkyl, C₁₋₂₀-Aminoalkyl, C₂₋₂₀- Hydroxyalkylaminoalkyl, C₂₋₃₀-Alkoxyalkyl, C₃₋₃₀-Dialkylaminoalkyl, C₂₋₃₀-Alkylaminoalkyl, R⁵-(OCR⁶R⁷CR⁸R⁹)ₙ-(OCR⁶R⁷), Aryl, Heteroaryl, C₇₋₂₀-Aralkyl, C₄₋₂₀-Heteroarylalkyl, C₇₋₂₀-Alkylaryl, C₄₋₂₀-Alkylheteroaryl, und Y-(CH₂)ₘ-NR⁵-(CH₂)_{q} oder gemeinsam -(CH₂)ₗ-X-(CH₂)ₘ- oder
R² und R⁴ gemeinsam -(CH₂)ₗ-X-(CH₂)ₘ-,
R⁵, R¹⁰ Wasserstoff (H), C₁₋₄-Alkyl, C₇₋₄₀-Alkylphenyl,
R⁶, R⁷, R⁸, R⁹ Wasserstoff (H), Methyl oder Ethyl,
X CH₂, CHR⁵, Sauerstoff (O), Schwefel (S) oder NR⁵,
Y N(R¹⁰)₂, Hydroxy, C₂₋₂₀-Alkylaminoalkyl oder C₃₋₂₀-Dialkylaminoalkyl,
n eine ganze Zahl von 1 bis 30 und
j, k, l, m, q eine ganze Zahl von 1 bis 4
bedeuten, durch Umsetzung eines primären oder sekundären Alkohols der Formel II 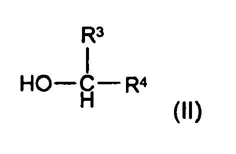 oder Aldehyds oder Ketons der Formel VI bzw. VII 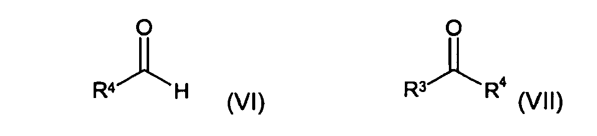 mit einer Stickstoffverbindung der Formel III

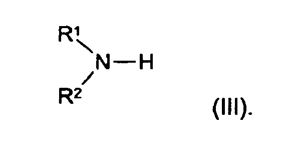

## Claims

1. A process for preparing an amine by reacting a primary or secondary alcohol, aldehyde or ketone with hydrogen and a nitrogen compound selected from the group consisting of ammonia and primary and secondary amines in the presence of a catalyst whose preparation has involved precipitation of catalytically active components onto monoclinic or tetragonal zirconium dioxide, the catalytically active composition of the catalyst before treatment with hydrogen comprises
from 20 to 65% by weight of oxygen-containing compounds of zirconium, calculated as ZrO₂,
from 1 to 30% by weight of oxygen-containing compounds of copper, calculated as CuO,
from 15 to 50% by weight of oxygen-containing compounds of nickel, calculated as NiO, and
from 15 to 50% by weight of oxygen-containing compounds of cobalt, calculated as CoO.

2. The process according to the preceding claim, wherein the metal salts are basic salts which are sparingly soluble or insoluble in water.

3. The process according to either of the two preceding claims, wherein the salts are oxides, hydrated oxides, hydroxides, carbonates and/or hydrogencarbonates.

4. The process according to claim 1, wherein the molar ratio of nickel to copper is greater than 1.

5. The process according to any of the preceding claims, wherein the monoclinic or tetragonal zirconium dioxide comprises one or more oxides of metals of transition group IIIB or main group IIA of the Periodic Table.

6. The process according to any of the preceding claims, wherein the reaction is carried out at from 80 to 300°C.

7. The process according to any of the preceding claims, wherein the reaction is carried out in the liquid phase at pressures of from 5 to 30 MPa or in the gas phase at pressures of from 0.1 to 40 MPa.

8. The process according to any of the preceding claims for preparing an amine of the formula I
where 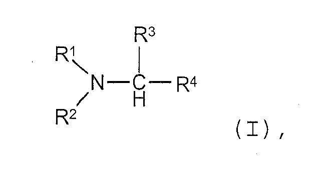
R¹, R² are each hydrogen (H), C₁₋₂₀-alkyl, C₃₋₁₂-cycloalkyl, C₂₋₃₀-alkoxyalkyl, C₃₋₃₀-dialkylaminoalkyl, aryl, C₇₋₂₀-aralkyl, C₇₋₂₀-alkylaryl, or together form - (CH₂)ⱼ-X-(CH₂)ₖ-,
R³, R⁴ are each hydrogen (H), C₁₋₂₀₀-alkyl, C₃₋₁₂-cycloalkyl, C₁₋₂₀-hydroxyalkyl, C₁₋₂₀-aminoalkyl, C₂₋₂₀- hydroxyalkyl-aminoalkyl, C₂₋₃₀-alkoxyalkyl, C₃₋₃₀-dialkylaminoalkyl, C₂₋₃₀-alkylaminoalkyl, R⁵-(OCR⁶R⁷CR⁸R⁹)ₙ-(OCR⁶R⁷), aryl, heteroaryl, C₇₋₂₀-aralkyl, C₄₋₂₀-heteroarylalkyl, C₇₋₂₀-alkylaryl, C₄₋₂₀-alkylheteroaryl or Y- (CH₂)ₘ-NR⁵-(CH₂)_{q}, or together form -(CH₂)ₗ-X-(CH₂)ₘ-, or
R² and R⁴ together form - (CH₂)ₗ-X-(CH₂)ₘ-,
R⁵, R¹⁰ are each hydrogen (H), C₁₋₄-alkyl or C₇₋₄₀-alkylphenyl,
R⁶, R⁷, R⁸, R⁹ are each hydrogen (H), methyl or ethyl,
X is CH₂, CHR⁵, oxygen (O), sulfur (S) or NR⁵,
Y is N(R¹⁰)₂, hydroxy, C₂₋₂₀-alkylaminoalkyl or C₃₋₂₀-dialkylaminoalkyl,
n is an integer from 1 to 30 and
j, k, l, m, q are each an integer from 1 to 4,
by reacting a primary or secondary alcohol of the formula II 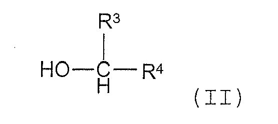 or aldehyde or ketone of the formula VI or VII 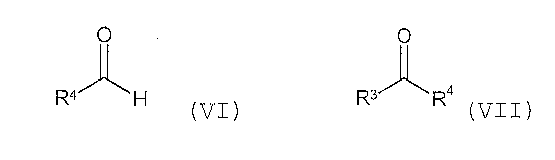 with a nitrogen compound of the formula III

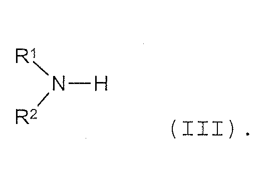

## Revendications

1. Procédé de préparation d'une amine par réaction d'un alcool primaire ou secondaire, d'un aldéhyde ou d'une cétone avec de l'hydrogène et un composé azoté choisi dans le groupe formé par l'ammoniac, des amines primaires et secondaires, en présence d'un catalyseur, **caractérisé en ce que** l'on met en oeuvre un catalyseur au cours de la préparation duquel a lieu une précipitation de composants à activité catalytique sur du dioxyde de zirconium monoclinique ou tétragonal, la masse active du catalyseur contenant, avant le traitement à l'hydrogène,
de 20 à 65 % en poids de composés oxygénés du zirconium, calculés en ZrO₂,
de 1 à 30 % en poids de composés oxygénés du cuivre, calculés en CuO,
de 15 à 50 % en poids de composés oxygénés du nickel, calculés en NiO, et
de 15 à 50 % en poids de composés oxygénés du cobalt, calculés en CoO.

2. Procédé suivant la revendication qui précède, **caractérisé en ce que** les sels métalliques sont des sels basiques peu solubles ou insolubles dans l'eau.

3. Procédé suivant l'une des deux revendications qui précèdent, **caractérisé en ce que** les sels sont des oxydes, des hydrates d'oxydes, des hydroxydes, des carbonates et/ou des hydrogénocarbonates.

4. Procédé suivant la revendication 1, **caractérisé en ce que** la proportion molaire du nickel au cuivre est supérieure à 1.

5. Procédé suivant l'une quelconque des revendications qui précèdent, **caractérisé en ce que** le dioxyde de zirconium monoclinique ou tétragonal contient un ou plusieurs oxydes de métaux du sous-groupe IIIB ou du groupe principal IIA du système périodique.

6. Procédé suivant l'une quelconque des revendications qui précèdent, **caractérisé en ce que** l'on entreprend la réaction à des températures de 80 à 300 °C.

7. Procédé suivant l'une quelconque des revendications qui précèdent, **caractérisé en ce que** l'on entreprend la réaction en phase liquide, à des pressions de 5 à 30 MPa ou en phase gazeuse à des pressions de 0,1 à 40 MPa.

8. Procédé suivant l'une quelconque des revendications qui précèdent pour la préparation d'une amine de la formule I 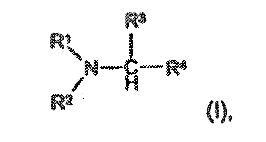 dans laquelle
R¹, R² représentent de l'hydrogène (H) ou un groupe alkyle en C₁ à C₂₀, cycloalkyle en C₃ à C₁₂, alcoxyalkyle en C₂ à C₃₀, dialkylaminoalkyle en C₃ à C₃₀, aryle, aralkyle en C₇ à C₂₀, alkylaryle en C₇ à C₂₀ ou représentent ensemble -(CH₂)ⱼ-X-(CH₂)ₖ-,
R³, R⁴ représentent de l'hydrogène (H) ou un groupe alkyle C₁ à C₂₀, cycloalkyle en C₃ à C₁₂, hydroxyalkyle en C₁ à C₂₀, aminoalkyle en C₁ à C₂₀, hydroxyalkylaminoalkyle en C₂ à C₂₀, alcoxyalkyle en C₂ à C₃₀, dialkylaminoalkyle en C₃ à C₃₀, alkylaminoalkyle en C₂ à C₃₀, R⁵- (OCR⁶R⁷CR⁸R⁹)ₙ-(OCR⁶R⁷), aryle, hétéroaryle, aralkyle en C₇ à C₂₀, hétéroarylalkyle en C₄ à C₂₀, alkylaryle en C₇ à C₂₀, alkylhétéroaryle en C₄ à C₂₀, et Y-(CH₂)ₘ-NR⁵-(CH₂)_{q} ou représentent ensemble -(CH₂)ₗ-X-(CH₂)ₘ-, ou
R² et R⁴ représentent ensemble -(CH₂)ₗ-X-(CH₂)ₘ-,
R⁵, R¹⁰ représentent de l'hydrogène (H) ou un groupe alkyle en C₁ à C₄, alkylphényle en C₇ à C₄₀,
R⁶, R⁷, R⁸, R⁹ représentent de l'hydrogène (H) ou un groupe méthyle ou éthyle,
X représente CH₂, CHR⁵, de l'oxygène (O), du soufre (S) ou NR⁵,
Y représente N(R¹⁰)₂ ou un groupe hydroxy, alkylaminoalkyle en C₂ à C₂₀ ou dialkylaminoalkyle en C₃ à C₂₀,
n est un nombre entier de 1 à 30 et
j, k, l, m, q sont des nombres entiers de 1 à 4,
par réaction d'un alcool primaire ou secondaire de formule II 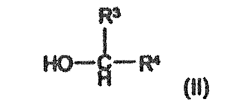 ou d'un aldéhyde ou d'une cétone de formules VI ou VII 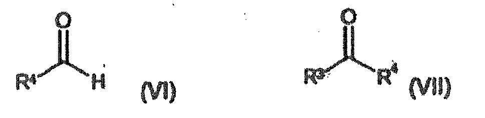 avec un composé azoté de formule III
